# DEMANDE DE BREVET EUROPEEN

(11) **EP 1 559 396 A1**
(43) Date de publication de la demande: **03.08.2005**
(21) Numéro de dépôt: 05290110.5
(22) Date de dépôt: 19.01.2005
(51) Int. Cl.: A61K 7/06, A61K 7/48

(54) **Procédé de préparation d'une composition pour le traitement cosmétique des matiéres kératiniques à partir de fluide sous pression et de composés cosmétiquement actifs liquids piegés par des particules**

(30) Priorité: 29.01.2004 FR 0400855
(71) Demandeur: L'OREAL, 75008 Paris (FR)
(72) Inventeur: De La Mettrie, Roland, 78110 Le Vesinet (FR); Grollier, Jean-Francois, 75006 Paris (FR); Cotteret, Jean, 78480 Verneuil Sur Seine (FR)
(74) Mandataire: Casalonga, Axel

(57) **Abrégé**

L'invention concerne un procédé de préparation d'une composition pour le traitement cosmétique des matières kératiniques, caractérisé en ce qu'il comprend une étape de percolation d'un fluide sous une pression d'au moins 3 bars au travers d'au moins composé cosmétiquement actif à l'état liquide piégé par des particules poreuses ou traitées en surface.

## Description

La présente invention a pour objet un procédé de préparation d'une composition cosmétique destinée au traitement des matières kératiniques des êtres humains, en particulier de la peau et des fibres kératiniques humaines telles que les cheveux ou les ongles, ainsi qu'un procédé de traitement cosmétique des matières kératiniques à partir de cette composition.

En cosmétique, on cherche toujours à améliorer les propriétés cosmétiques des matières kératiniques, par exemple des cheveux sensibilisés, i.e. qui sont abîmés ou fragilisés sous l'action chimique d'agents extérieurs comme les agents atmosphériques, l'eau de mer ou de piscine et/ou des traitements capillaires tels que permanentes, défrisages, teintures ou décolorations. On peut alors leur appliquer des compositions de traitement cosmétique comprenant des composés cosmétiquement actifs à l'état liquide, comme des agents de conditionnement, tels que des huiles, destinés à réparer ou limiter les effets néfastes ou indésirables produits par les différents traitements ou agressions que subissent, de manière plus ou moins répétée, les matières kératiniques. Ces agents de conditionnement peuvent également améliorer les propriétés cosmétiques comme l'aspect ou le toucher des cheveux naturels. Ces compositions de traitement peuvent être appliquées avant ou après l'action des agents atmosphériques et/ou des traitements.

Il est également connu d'utiliser des extraits végétaux pour traiter la peau, et notamment pour obtenir des propriétés adoucissantes, anti-inflammatoires, antiprurigineuses, antiseptiques, antisudorales, astringentes, calmantes, cicatrisantes, raffermissantes, parfumantes et/ou tonifiantes mais aussi pour nourrir ou hydrater la peau, la protéger des agents extérieurs, éliminer les peaux mortes, diminuer les rides et ridules ainsi que les taches colorées (défaut de pigmentation ou taches apparaissant avec l'age en particulier sur les mains, le cou ou le visage) ou encore colorer la peau.

Ainsi, on utilise pour leurs propriétés texturantes : le blé, le fucus ; pour leurs propriétés antioxydantes : le riz, le romarin, la sauge, le thym, le thé vert, le réglisse ; pour leurs propriétés moussantes ou émulsifiantes : la saponaire, le lierre, le fragon, le bois de panama, le quillaja, la salsepareille, le quino, le soja ; pour leurs propriétés photoprotectrices : l'aloes, le tournesol, le réglisse, le magnolia, le kaemperia ; pour leurs propriétés épaississantes ou absorbantes : les pois, le blé, les pommes de terre, le maïs ; pour leurs propriétés odorantes : le romarin , la violette, la lavande, la rose.

Il est connu différents traitements de composés cosmétiquement actifs.

Le document US 5 895 672 décrit un système pour la production de thé, et notamment de thé vert, utilisant la technologie expresso.

Le document US 6 468 564 décrit des compositions pour le traitement de la peau à base d'extraits de plantes.

Le document US 6 146 616 décrit des compositions anti-oxydantes à base d'huile de lupin, notamment pour le traitement de la peau.

Le document US 2003/096038 décrit une cartouche pour la préparation de boissons. La boisson est obtenue par le passage d'eau sous pression à travers de composés solides.

Le document US 2 526 607 décrit un procédé de production de cire à partir de bois comprenant l'hydrolyse de la cellulose contenue dans le bois à l'aide d'un acide.

La demande FR 2 207 699 décrit un procédé de purification d'extraits végétaux contenant des pigments flavoniques.

La demande EP 0 384 796 décrit un procédé d'obtention d'extraits riches en pyonogénols à partir de matière première végétale.

La demande WO 03/095979 décrit un procédé de traitement d'échantillons botaniques à l'aide de vapeur d'eau sous pression.

Le document US 5 405 633 décrit un procédé d'extraction d'huiles et de matières grasses à partir de produits naturels à l'aide de propane liquide comme solvant.

La demande EP 1 426 028 décrit un procédé de contrôle d'extraction de tourbe à l'aide de dioxyde de carbone.

La demande EP 0 464 297 décrit un procédé de préparation d'extraits lipophiles de plantes à partir de plantes à l'état solide.

Le document US 2003/157183 décrit un procédé d'encapsulation de fines particules solides sous la forme de microcapsules.

Aucun de ces documents n'enseigne de percolation de fluide au travers de composés cosmétiquement actifs à l'état liquide piégés par des particules solides.

Cependant, les compositions de traitement cosmétique contenant ces composés cosmétiquement actifs sont généralement des compositions aqueuses dans lesquelles lesdits agents doivent être solubilisés. Le manque de solubilité de ces composés diminue le caractère traitant de ces compositions. En outre, ce critère de solubilité réduit le nombre de composés cosmétiquement actifs que l'on peut utiliser pour le traitement cosmétique des matières kératiniques ainsi que le nombre de compositions les contenant que l'on peut offrir aux consommateurs. Ceci est particulièrement le cas pour des composés à point de fusion élevé.

Par ailleurs, ces composés cosmétiquement actifs sont généralement instables en solution aqueuse et/ou en milieu atmosphérique, ou encore en présence de certains réactifs chimiques comme les agents réducteurs ou oxydants, ce qui conduit à des compositions cosmétiques peu efficaces et/ou d'aspect, d'odeur et/ou de toucher inacceptables pour le consommateur, en quelques jours.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en composé cosmétiquement actif, selon le besoin, notamment sans conservateur, permettant de surmonter les problèmes de solubilité exposés ci-dessus.

Ce procédé est mis en oeuvre simplement et est approprié au besoin du consommateur en un actif ou en mélange d'actifs. Le composé ou mélange de composés cosmétiquement actifs à l'état liquide est piégé par des particules appropriées et notamment poreuses ou traitées en surface, et on fait passer un fluide sous pression, dont la température est supérieure à la température ambiante (20-25°C à pression atmosphérique (10⁵ Pa)) et de préférence supérieure à 30°C, pendant un laps de temps très court, inférieur à une minute, au travers du composé ou mélange de composés cosmétiquement actifs piégés par ces particules en particulier poreuses ou traitées en surface.

Il permet d'utiliser sous forme anhydre des composés cosmétiquement actifs instables dans des compositions aqueuses, soit par réaction avec l'eau ou l'humidité ambiante, soit par réaction en solution aqueuse avec des composés qui ne réagissent pas entre eux en milieu anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement de la composition jusqu'à une température acceptable pour les matières kératiniques, notamment inférieure à 60°C et mieux inférieure à 50°C. La composition peut être utilisée jusqu'à une semaine après sa préparation, selon la vitesse de dégradation du composé cosmétiquement actif ou du mélange de composés cosmétiquement actifs.

Etant donné le temps de préparation très court, les compositions de traitement cosmétique peuvent être préparées "à la demande" en mélangeant les différents composés cosmétiquement actifs selon les propriétés cosmétiques recherchées.

Selon un autre mode de réalisation, les composés cosmétiquement actifs peuvent être conditionnés dans un dispositif prêt-à-l'emploi, et il n'est pas nécessaire de déterminer au préalable les concentrations des composés actifs en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions conférant de bonnes propriétés cosmétiques.

L'invention a donc pour objet un procédé de préparation d'une composition de traitement cosmétique des matières kératiniques comprenant une étape de percolation d'un fluide sous une pression d'au moins 3 bars au travers d'au moins un composé cosmétiquement actif piégé par des particules solides notamment poreuses ou traitées en surface.

Un autre objet de l'invention est une composition susceptible d'être obtenue par le procédé selon l'invention.

L'invention a également pour objet l'utilisation notamment cosmétique de la composition obtenue selon le procédé de l'invention, comme agent pour le traitement cosmétique des matières kératiniques.

L'invention a enfin pour objet un dispositif de conditionnement permettant de mettre en oeuvre le procédé de préparation de la présente invention.

L'invention a aussi pour objet un procédé de traitement cosmétique des matières kératiniques des êtres humains, comprenant a) la préparation d'une composition cosmétique prête à l'emploi par percolation d'un fluide sous pression d'au moins d'au moins 3 bars au travers d'au moins un composé cosmétiquement actif piégé par des particules solides poreuses ou traitées en surface et b) l'application de la composition obtenue à l'étape a) aux matières kératiniques.

L'application aux matières kératiniques peut, selon la nature des actifs, être réalisée par voie topique ou voie orale, et de préférence par voie topique.

D'autres objets, caractéristiques, aspects et avantages de l'invention apparaîtront encore plus clairement à la lecture de la description et des exemples qui suivent.

Par composé cosmétiquement actif, on entend tout composé ayant une activité cosmétique ou dermatologique, à savoir un composé ayant une action bénéfique pour la peau ou les fibres kératiniques.

Selon l'invention, le procédé de préparation d'une composition de traitement cosmétique des matières kératiniques comprend une étape de percolation d'un fluide de préférence à une température au moins égale à 30°C et par exemple comprise entre 30°C et 150°C, mieux encore allant de 40 à 120 °C, sous une pression d'au moins 3 bars (3. 10⁵ Pa), au travers d'au moins un composé liquide cosmétiquement actif piégé par des particules solides poreuses ou traitées en surface.

Les notions de "liquide" et "solide" font référence à un état physique du composé actif ou des particules à une température de 20-25°C et une pression atmosphérique de 10⁵ Pa. En outre, les particules présentent avantageusement une température de fusion élevée, à savoir au moins supérieure à la température de percolation utilisée.

La percolation est un mouvement de fluide à travers un milieu poreux saturé permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par « composé insoluble dans l'eau », on entend tout composé qui, à une concentration supérieure ou égale à 0,1% en poids dans l'eau à 25°C, ne forme pas à l'oeil nu une solution isotrope transparente.

Le composé cosmétiquement actif à l'état liquide est piégé par des particules solides ou immobilisé sur des particules solides, ces particules étant capables d'adsorber ou absorber ledit composé ; ces particules sont notamment poreuses ou traitées en surface. Par « particules traitées en surface », on entend des particules qui ont été traitées de façon à pouvoir accrocher par exemple par adsorption le composé cosmétiquement actif.

Le piégeage du composé cosmétiquement actif à l'état liquide peut s'effectuer par mise en contact du composé cosmétiquement actif avec les particules solides poreuses ou traitées en surface, à l'aide de nombreuses techniques connues de l'homme du métier. Parmi celles-ci, on peut citer l'imprégnation, la lyophilisation, notamment décrite dans le brevet FR 2 099 049, le séchage par pulvérisation, ou encore l'atomisation.

Par matières kératiniques, on entend la peau, le cuir chevelu, les lèvres, et/ou les phanères tels que les ongles, les cils, les sourcils et les cheveux.

Le procédé de la présente invention peut être mis en oeuvre à partir d'un dispositif classique permettant de générer un fluide sous pression, à une température supérieure à la température ambiante, de préférence au moins égale à 30°C et par exemple comprise entre 30°C et 150°C, et encore plus préférentiellement allant de 40°C à 120°C. Un tel dispositif comprend une chambre résistant à la pression, équipée d'un bloc thermique, ainsi qu'un circuit d'acheminement du fluide produit vers le composé cosmétiquement actif piégé.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type « expresso ». De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température au moins égale à 30°C et notamment allant de 30°C à 150°C, de préférence allant de 40°C à 120°C, sous une pression comprise entre 3 et 30 bars, ou d'au moins 4 bars, de préférence au moins égale à 10 bars et tout particulièrement comprise entre 10 et 30 bars.

Le ou les composés cosmétiquement actifs piégés par des particules solides (ou immobilisés sur ces particules) peuvent être utilisés directement, dans le dispositif générant le fluide sous pression, dans un récipient destiné à cet usage. Ils peuvent également être conditionnés dans un dispositif de conditionnement particulier du type monodose comprenant un logement fermé délimité par au moins une paroi au moins en partie perméable à un fluide sous pression d'au moins 3 bars. De tels dispositifs sont, par exemple, décrits dans les demandes de brevets WO00/56629, EP512470, US5897899 ou WO99/03753. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple, en cellulose, en métal tel que l'aluminium ou en composite. De tels dispositifs sont par exemple décrits dans les demandes de brevets WO00/56629, EP512470, US5897899 ou WO99/03753.

Le composé cosmétiquement actif à l'état liquide ou le mélange de composés cosmétiquement actifs peut être choisi parmi les extraits aqueux ou hydroalcooliques que l'on peut piéger ou immobiliser sur des particules solides notamment poreuses ou traitées en surface.

Les particules solides poreuses ou traitées en surface sont en particulier inertes chimiquement (à savoir ne réagissent pas chimiquement avec le ou les actifs qu'elles piègent) ; elles peuvent être minérales ou organiques, lamellaires, sphériques ou oblongues. Elles sont par exemple choisies parmi les particules d'alumine, de zéolithes, de chitosane, de maltodextrine, de cyclodextrine, de carraghénane, d'oxyde de zinc ou de zirconium, de silice, kaolin, talc, carbonate de calcium précipité, carbonate ou hydroxycarbonate de magnésium, les particules creuses de résine notamment de silicone ou de polysiloxane, les microsphères de silice creuses ( SilicaBeads® de Maprecos), le coton, les composés superabsorbants, l'amidon, éventuellement modifié, les grains ou farines de maïs, de blé, de riz, d'orge, de pois, l'argile, la sciure de bois, l'alginate de calcium ou d'oligosaccharides, le charbon actif, les particules creuses de poly(chlorure de vinylidène/acrylonitrile), notamment celles commercialisées sous la dénomination générale d'« Expancel® » par la société AKZO NOBEL sous les références particulières « Expancel® WE » ou « DE » Expancels, les poudres creuses de polyamide et notamment de Nylon (comme Orgasol® de chez Atochem), les particules poreuses de céramiques, l'hydroxyapatite poreuse, les particules creuses de verre, et leurs mélanges.

De préférence, on utilise des particules de Nylon, de silice poreuse, de maltodextrine, d'amidon, de carraghénane, d'Expancel, d'alginate de calcium ou d'oligosaccharides, de polysiloxane.

Le ou les composés cosmétiquement actifs à l'état liquide peuvent être choisis parmi :
- les extraits aqueux, hydroalcooliques ou huileux des végétaux comme les extraits d'hamamélis pour des propriétés astringentes et parfumantes, les extraits glycoliques de fleur de lys (actif parfumant), l'extrait d'iris florentina (actif anti-age), l'extrait de pimprenelle pour le traitement des peaux irritées (par exemple par le rasage, le soleil), les acides de fruit (glycolique, lactique) comme desquamant, les extraits aqueux d'iris pallida (anti-irritant), l'extrait hydroglycolique de sauge (comme actif cicatrisant, anti-inflammatoire, amincissant), l'extrait de lycopène (notamment de tomate) comme anti-oxydant ;
- les acides gras essentiels insaturés, (comme par exemple l'acide linoléique ou linolénique), comme actif émollient, ou les alcools gras essentiels insaturés (comme par exemple l'alcool oléique, linolénique) à action émolliente ;
- les huiles non volatiles, comme par exemple les huiles végétales insaturées contenant généralement des esters insaturés (de soja, d'olive, de pépins de raisin, de coriandre) à propriétés émollientes avec pour le coriandre des propriétés anti-inflammatoires ;
- les parfums ( connus pour leur instabilité à la lumière) et les huiles essentielles ;
- l'eau oxygénée ;
- les agents réducteurs à l'état liquide comme l'acide thiolactique et ses esters (notamment le monothiolactate de glycérol), l'acide thioglycolique ainsi que ses esters, notamment le monothioglycolate de glycérol ou de glycol, le dithiodiglycolate de diammonium ou le thioglycolate d'ammonium, et le thioglycérol ;
- les filtres solaires à l'état liquide, comme par exemple le Cinnoxate, l'éthylhexyl dimethyl PABA, l'éthylhexyl méthoxycinnamate, l'homosalate, le menthyl anthranilate, l'octocrylène, l'éthylhexyl salicylate, le PEG-25 PABA,
- les vitamines ou dérivés de vitamines à l'état liquide comme la vitamine A et ses esters tels que le palmitate de vitamine A, la vitamine E est ses esters comme l'acétate ou le palmitate de vitamine E,
- les esters liquides de mono ou de polyacides avec des mono ou polyols tels que le myristate d'isopropyle, l'isononanoate d'octyle ou de tridécyle,
- les huiles de silicone telles que les polydiméthylsiloxanes liquides volatiles et les cyclométhicones, la température du percolateur devant toutefois être pour les huiles de l'ordre de 30°C, de préférence comprise entre 30 et 40°C,
- les tensioactifs liquides comme les alcools gras oxyéthylénés liquides tels que le laureth 2, les mono ou trioléates de sorbitane oxyéthylénés.

Les huiles non volatiles mentionnées ci-dessus peuvent être choisies parmi celles utilisées classiquement en cosmétique. Par huile, on entend tout corps gras liquide à 25°C, sous 1 atm, et ayant un poids moléculaire supérieur ou égal à 160, notamment compris entre 170 et 10⁶, voire entre 200 et 5.10⁵, compatible avec une application sur la peau, les lèvres et/ou les phanères (ongles, cils, sourcils, cheveux).

Les huiles susceptibles d'être utilisées dans le procédé selon l'invention peuvent être, d'origine animale, végétale, minérale ou synthétique. Elles servent généralement pour le conditionnement des cheveux, la protection et la nutrition de la peau et/ou son émollience.

On peut notamment citer comme autres huiles que celles citées précédemment :
- les huiles d'origine végétale ou animale telles que les huiles de tournesol, d'onagre, de carthame, de passiflore, de seigle, de courge, de sésame, de noisette, de noyau d'abricot, d'amande ou d'avocat, de calophyllum, de macadamia, de colza, de coprah, d'arachide, de palme, de ricin, de jojoba, ou de germes de céréales telles que l'huile de germe de blé ou de maïs, l'huile de beurre de karité ; les huiles de poisson, le perhydrosqualène, le tricaprocaprylate de glycérol ;
- les huiles de synthèse comme les huiles de formule R₁COOR₂ dans laquelle R₁ représente le reste d'un acide gras supérieur comportant de 7 à 29 atomes de carbone et R₂ représente une chaîne hydrocarbonée contenant de 3 à 30 atomes de carbone comme, par exemple, l'huile de Purcellin, le myristate de butyle, le myristate d'isopropyle, le myristate de cétyle, le palmitate d'isopropyle, le palmitate de 2-éthylhexyle, l'adipate d'isopropyle, l'adipate d'éthylhexyle, le stéarate de butyle, le stéarate d'hexadécyle, le stéarate d'isopropyle, le stéarate d'octyle, le stéarate d'isocétyle, le stéarate de 2-octyldodécyle, l'oléate de décyle, le laurate d'hexyle, l'érucate de 2-octyldodécyle, l'isononanoate d'isononyle, l'isostéarate d'isostéaryle ; les esters hydroxylés comme le lactate d'isostéaryle, l'hydroxystéarate d'octyle, l'hydroxystéarate d'octyldodécyle, le malate de diisostéaryle, le citrate de triisocétyle ; des heptanoates, octanoates, décanoates d'alcools gras ; des esters de polyol comme les octanoates ou décanoates d'éthylèneglycol ou de glycérol, le dicaprylate de propylèneglycol, le dioctanoate de propylèneglycol, le diheptanoate de néopentylglycol, le diisononanoate de diéthylèneglycol ; les esters du pentaérythritol comme le tétraisostéarate de pentaérythrithyle ; des esters du type trimellitate de tridécyle ;
- des hydrocarbures linéaires ou ramifiés, d'origine minérale ou synthétique tels que les huiles de paraffine et leurs dérivés, les poly(α-oléfines), la vaseline, les polydécènes, les polyisobutènes hydrogénés tels que le Parléam ;
- des glycérides et notamment des acétylglycérides ou des triglycérides d'acides gras ayant 4 à 10 atomes de carbone, comme les triglycérides des acides heptanoïque, octanoïque et caprique/caprylique, les triglycérides d'acides gras saturé en C₁₀₋₁₈ ;
- les huiles fluorées et perfluorées, la lanoline, la lanoline hydrogénée et la lanoline acétylée.

De préférence, les actifs cosmétiquement actifs liquides utilisables dans l'inventions sont les huiles essentielles, les extraits liquides de végétaux et notamment d'iris pallida, de lycopène, de marc de raisin, les acides de fruits, l'extrait d'aloes, la DHA, les filtres, les vitamines liquides et leurs esters liquides, les esters de monoacides et de monoalcools.

Il est possible d'utiliser des dispersions de composés solides dans ces composés liquides et de pouvoir ainsi les adsorber sur ou de les adsorber dans un support solide éventuellement poreux.

Il est possible pour l'utilisateur d'utiliser des composés cosmétiquement actifs qui sont vendus déjà piégés sur des particules absorbantes et notamment poreuses. Par exemple, on peut citer l'extrait de mélisse atomisé sur des particules de maltodextrine ; des alpha-hydroxyacides liquides atomisés sur des particules de polysaccharides et/ou amidon (acides glucuronique/manuronique 50/50 en poudre atomisés sur des particules d'alginates d'oligosaccharides vendus et/ou fabriqués par la société Codif pour des propriétés apaisantes et/ou anti-inflammatoires) ; des particules de polyamide 12 imprégnées de dihydroxyacétone (DHA) (vendues et/ou fabriquées sous la dénomination Orgasol DHA ou Orgasol 2002 N5 HA par la société Atochem) et des microsphères de carraghénane imprégnées de DHA en dispersion huileuse (vendus et/ou fabriquées sous la dénomination DHA 2011 par la société Lipotec) pour la coloration de la peau ; de l'exsuda ou de la sève d'aloes pour des propriétés apaisantes, anti-irritantes pour la peau ou la beauté du cheveu, piégé sur des sphères d'agar-agar (Softsphere SP-VEAAV GRP 41 M de chez KOBO) ; du jus de sureau rouge concentré en poudre, atomisé sur maltodextrine (poudre de sureau de la société Diana Vegetal) ; de l'extrait de marc de raisin sur maltodextrine comme agent antiradicalaire ou anti-oxydant (par exemple les polyphénols de marc de raisin vendus sous la dénomination ACWS P 118 par la société Sefcal) ; des nano-sphères de polysiloxane chargées en palmitate de vitamine A ou les microsphères de polyamide 12 imprégnées de palmitate de vitamine A (Orgasol 2002 UD+10% vitamine A de chez Atochem) pour ses propriétés nourrisantes ou anti-inflammatoire des microbilles de silice poreuses remplies d'acétate de vitamine E (SB-700/VEAC de chez Miyoshi Kasei) pour ses propriétés hydratantes et anti-oxydantes.

Le ou les composés cosmétiquement actifs piégés selon l'invention peuvent être mis en oeuvre en mélange avec un ou plusieurs adjuvants solides ou pâteux, et de préférence pulvérulents. Les adjuvants peuvent être choisis parmi les argiles, les sels, les agents tensioactifs anioniques, cationiques, non-ioniques, amphotères, zwittérioniques, les épaississants naturels ou de synthèse, l'amidon éventuellement modifié, les billes de verre, la silice, le Nylon®, l'alumine, le dioxyde de titane, les zéolithes, le poly(méthylméthacrylate) (PMMA), le chitosane, la maltodextrine, la cyclodextrine, les mono- ou disaccharides comme le glucose, le saccharose, le sorbitol, le fructose, l'oxyde de zinc, de zirconium, les particules de résine notamment de silicone, le talc, l'acide polyaspartique, les borosilicates notamment de calcium, le polyéthylène, le polytétrafluoroéthylène (PTFE), la cellulose et ses esters, les composés superabsorbants, les carbonates de magnésium ou de calcium, les grains ou farines de maïs, de blé, de riz, de pois, de lupin, de soja, d'orge, les gommes de polydiméthylsiloxane, le polyacrylamide, la soie, le collagène, la sciure de bois, la poudre de fucus, de polyvinylpyrrolidone réticulée, de l'alginate de calcium, le charbon actif, les particules de poly(chlorure de vinylidène/acrylonitrile), notamment celles commercialisées sous la dénomination générale d' « Expancel® » par la société AKZO NOBEL sous les références particulières « Expancel® WE » ou « DE » Expancels, et leurs mélanges.

De préférence comme adjuvants solides ou pâteux utilisables dans l'invention, on peut citer la silice, l'Expancel, le Nylon, le PTFE, la cellulose, l'amidon.

Lorsqu'un ou plusieurs adjuvants sont présents, le ou les composés cosmétiquement actifs piégés de l'invention sont présents, dans la composition soumise à la percolation sous pression, de préférence en une quantité allant de 0,5 à 99% en poids, mieux encore de 1 à 80 % en poids, et encore plus préférentiellement de 2 à 60 % en poids par rapport au poids total composé(s) cosmétiquement actif(s) piégés et adjuvants.

Les plantes ou extraits de plantes utilisés et au travers desquels passe le fluide sous pression peuvent être soumis avant la percolation à un traitement tel qu'une torréfaction, un cryobroyage, ou une lyophilisation.

La composition obtenue selon le procédé de l'invention contient outre le ou les composés cosmétiquement actifs piégés et le(s) composant(s) du fluide, à savoir l'eau et/ou le(s) solvant(s) cosmétiquement acceptable(s), éventuellement tout ou partie du ou des adjuvants présents dans le mélange solide ou pâteux, selon la nature chimique de ces adjuvants, et notamment leur aptitude à être entraînés par le fluide sous pression, et leur taille.

L'invention concerne également une composition susceptible d'être obtenue par le procédé selon l'invention, la composition étant de préférence exempte d'agents conservateurs.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité du ou des composés cosmétiquement actif piégés présents dans la composition finale de traitement cosmétique obtenue par le procédé de la présente invention est en général comprise dans l'intervalle allant de 0,001 à 50 % en poids environ du poids total de la composition finale de traitement cosmétique, de préférence de 0,005 à 30 %, et encore plus préférentiellement de 0,01 à 20%.

Lorsque la composition cosmétique obtenue par le procédé de percolation de la présente invention est mélangée à un milieu cosmétiquement acceptable, le milieu est généralement constitué par de l'eau ou par un mélange d'eau et d'au moins un solvant organique pour solubiliser les composés qui ne seraient pas suffisamment solubles dans l'eau. Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40% en poids par rapport au poids total de la composition finale, et encore plus préférentiellement entre 5 et 30% en poids.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement différents de ceux décrits ci-dessus tels que, par exemple, des huiles de silicone, des agents filmogènes, des céramides, des agents conservateurs, des agents opacifiants, mais aussi des huiles, des cires, des gommes, des pigments colorés ou nacrés.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés de manière telle que les propriétés avantageuses attachées intrinsèquement à la composition cosmétique conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 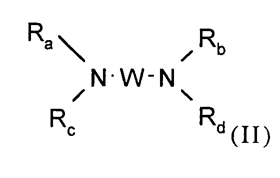 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition finale de traitement cosmétique peut se présenter sous des formes diverses, telles que sous forme de liquides, de crèmes, de gels, de mascara ou sous toute autre forme appropriée pour réaliser un traitement des matières kératiniques, et notamment des fibres kératiniques, et de la peau. Cette composition finale peut être utilisée pour le traitement et/ou le soin du cheveu et/ou le traitement et/ou le soin et/ou le maquillage de la peau, des ongles, des cils ou encore des lèvres.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

La quantité totale du ou des composés cosmétiquement actifs piégés sur des particules solides, éventuellement associés à un ou plusieurs adjuvants pateux ou solides, est fontion de la taille de la chambre de percolation ou du conditionnement étanche utilisé ainsi que de la densité des ingrédients placés dans la chambre de percolation ou dans le dispositif de conditionnement monodose. En pratique cette quantité varie de 1g à 10g et par exemple de 2 à 5g.

L'exemple ci-après est destiné à illustrer la présente invention.

### Exemple 1

Dans cet exemple, on utilise une machine à expresso du commerce dont le volume de la chambre est de 13ml.

On prépare 5g en poudre de microsphères de carraghénane imprégnées de dihydroxyacétone en dispersion huileuse, vendues sous la dénomination DHA 2011 par la société Lipotec.

On place ce mélange dans la machine expresso du commerce telle que celle décrite dans les documents cités ci-dessus. On fait ensuite passer de la vapeur d'eau jusqu'à l'obtention d'une composition (A) présentant un volume final de 50 ml.

On ajoute ensuite à deux parties en poids de composition (A), une partie en poids d'une composition aqueuse (B) contenant 1 % en poids d'hydroxyéthylcellulose.

On obtient ainsi une composition de coloration prête à être appliquée sur la peau.

### Exemple 2

On place 5g de micro-billes de silice poreuses remplies d'acétate de vitamine E et 4g de bicarbonate d'ammonium dans une machine expresso du commerce. On fait ensuite passer de la vapeur d'eau jusqu'à l'obtention d'une composition présentant un volume final de 50 ml.

On obtient ainsi une composition d'après shampoing prête à être appliquée sur les cheveux pour les nourrir et les conditionner.

## Revendications

1. Procédé de préparation d'une composition pour le traitement cosmétique des matières kératiniques, **caractérisé en ce qu'**il comprend une étape de percolation d'un fluide sous une pression d'au moins 3 bars au travers d'au moins un composé cosmétiquement actif à l'état liquide piégé par des particules solides.

2. Procédé selon la revendication 1, **caractérisé en ce que** les particules solides sont poreuses ou traitées en surface.

3. Procédé selon la revendication 1 ou 2, **caractérisé en ce que** le piégeage s'effectue par mise en contact du ou des composés cosmétiquement actifs, à l'état liquide, avec lesdites particules.

4. Procédé selon la revendication 3, **caractérisé en ce que** le piégeage est effectué par imprégnation, lyophilisation, séchage par pulvérisation ou atomisation.

5. Procédé selon l'une quelconque des revendications précédentes, **caractérisé en ce que** les particules solides sont choisies parmi les particules d'alumine, de zéolithes, de chitosane, de maltodextrine, de carraghénane, de cyclodextrine, d'oxyde de zinc ou de zirconium, de silice, kaolin, talc, carbonate de calcium précipité, carbonate ou hydroxycarbonate de magnésium, les particules creuses de résine, le coton, les composés superabsorbants, l'amidon, éventuellement modifié, les grains ou farines de maïs, de blé, de riz, de pois, d'orge, l'argile, la sciure de bois, , l'alginate de calcium ou d'oligosaccharides, le charbon actif, les particules de poly(chlorure de vinylidène/acrylonitrile), les poudres creuses de polyamide, les particules poreuses de céramiques, l'hydroxyapatite poreuse, les particules creuses de verre et leurs mélanges.

6. Procédé selon l'une quelconque des revendications précédentes, **caractérisé en ce que** le ou les composés cosmétiquement actif est choisi parmi les extraits de végétaux, les acides gras essentiels insaturés, les huiles non volatiles, les parfums, les huiles essentielles, l'eau oxygénée, les agents réducteurs à l'état liquide, les filtres solaires à l'état liquide, les vitamines ou dérivés de vitamines à l'état liquide, les esters liquides de mono ou de polyacides avec des mono ou polyols, les huiles de silicone, les tensioactifs liquides.

7. Procédé selon la revendication 6, **caractérisé en ce que** les extraits végétaux sont choisis parmi les extraits d'hamamélis, les extraits glycoliques de fleur de lys, l'extrait d'iris florentina, l'extrait de pimprenelle, les acides de fruit, les extraits aqueux d'iris pallida, l'extrait hydroglycolique de sauge, l'extrait de lycopène.

8. Procédé selon l'une quelconque des revendications précédentes, **caractérisé en ce que** le ou les composés cosmétiquement actifs piégés sont mis en oeuvre en mélange avec au moins un adjuvant.

9. Procédé selon la revendication 8, **caractérisé en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le Nylon, l'alumine, le dioxyde de titane, les zéolithes, le poly(méthacrylate de méthyle) (PMMA), le chitosane, la maltodextrine, la cyclodextrine, les mono-ou disaccharides, l'oxyde de zinc, de zirconium, les particules de résine, le talc, l'acide polyaspartique, les borosilicates notamment de calcium, le polyéthylène, le polytétrafluoroéthylène (PTFE), la cellulose et ses esters, les composés superabsorbants, les carbonates de magnésium ou de calcium, les grains ou farines de maïs, de blé, de riz, de pois, de lupin, de soja, d'orge, les gommes de polydiméthylsiloxane, le polyacrylamide, la soie, le collagène, la sciure de bois, la poudre de fucus, la polyvinylpyrrolidone réticulée, l'alginate de calcium, le charbon actif, les particules de poly(chlorure de vinylidène/acrylonitrile), et leurs mélanges.

10. Procédé selon la revendication 8 ou 9, **caractérisé en ce que** le ou les composé cosmétiquement actifs piégés sont présents en une quantité allant de 0,5 à 99% en poids, de préférence de 1 à 80 % et encore plus préférentiellement de 2 à 60% en poids par rapport au poids total de composé(s) cosmétiquement actif(s) piégé(s) et adjuvant.

11. Procédé selon l'une quelconque des revendications précédentes, **caractérisé en ce que** l'étape de percolation est mise en oeuvre avec de la vapeur d'eau sous une pression comprise entre 3 et 30 bars, de préférence entre 10 et 30 bars.

12. Procédé selon l'une quelconque des revendications précédentes, **caractérisé en ce que** l'étape de percolation est mise en oeuvre avec de la vapeur d'eau sous pression d'au moins 10 bars.

13. Composition de traitement cosmétique susceptible d'être obtenue par la procédé selon l'une quelconque des revendications précédentes.

14. Composition selon la revendication 13, **caractérisée en ce qu'**elle est exempte d'agents conservateurs.

15. Procédé de traitement cosmétique des matières kératiniques, **caractérisé en ce qu'**on prépare une composition cosmétique selon le procédé défini selon l'une quelconque des revendications 1 à 12 et qu'on applique cette composition sur la matière kératinique.

16. Procédé de traitement cosmétique des matières kératiniques selon la revendication 15, **caractérisé en ce qu'**on applique la composition sur la matière kératinique par l'intermédiaire d'un dispositif ne nécessitant pas d'intervention humaine.

17. Procédé de traitement cosmétique des matières kératiniques selon la revendication 15, **caractérisé en ce que**, avant l'application, la composition de traitement cosmétique préparée selon le procédé selon l'une quelconque des revendications 1 à 12, est mélangée à un milieu cosmétiquement acceptable et/ou à un ou plusieurs additifs utilisés en cosmétique.

18. Procédé de traitement cosmétique des matières kératiniques, **caractérisé en ce que** l'on prépare au moins deux compositions cosmétiques selon le procédé défini selon l'une quelconque des revendications 1 à 12, on les mélange et on applique le mélange sur la matière kératinique.

19. Utilisation d'une composition obtenue par le procédé selon l'une quelconque des revendications 1 à 12, comme agent pour le traitement et/ou le soin cosmétique des matières kératiniques.

20. Dispositif de conditionnement d'un composé cosmétiquement actif à l'état liquide piégé par des particules solides notamment poreuses ou traitées en surface, comprenant un logement fermé délimité par au moins une paroi au moins en partie perméable à un fluide sous pression d'au moins 3 bars, le logement contenant au moins un composé cosmétiquement actif à l'état liquide piégé par des particules poreuses ou traitées en surface.

21. Dispositif selon la revendication 20, **caractérisé en ce que** le logement est délimité par deux feuilles scellées.

22. Dispositif selon la revendication 21, **caractérisé en ce que** le logement est délimité par une barquette fermée par un couvercle.
